# EUROPÄISCHE PATENTSCHRIFT

(11) **EP 2 308 458 B1**
(45) Veröffentlichungstag und Bekanntmachung des Hinweises auf die Patenterteilung: **08.08.2018**
(21) Anmeldenummer: 10015597.7
(22) Anmeldetag: 12.03.2009
(51) Int. Cl.: A61K 8/41, A61K 8/34, A61K 8/36, A61Q 17/04, A61Q 19/00

(54) **ZUBEREITUNG FÜR DIE TAGESPFLEGE ENTHALTEND ÖLSÄURE**
PREPARATION FOR DAILY CARE CONTAINING OLEIC ACID
PRÉPARATION POUR SOIN DE JOUR CONTENANT DE L'ACIDE OLÉIQUE

(30) Priorität: 11.04.2008 DE 102008018787
(43) Veröffentlichungstag der Anmeldung: 13.04.2011
(62) Teilanmeldung aus: 09731286.2
(73) Patentinhaber: Beiersdorf Aktiengesellschaft, 20253 Hamburg (DE)
(72) Erfinder: Lerg, Heike, 22303 Hamburg (DE); Ruppert, Stephan, 20259 Hamburg (DE); Fey, Sven, 22397 Hamburg (DE); Blohm, Alexandera, 20257 Hamburg (DE); Espel, Anja, 22041 Hamburg (DE)

(56) Entgegenhaltungen:
- WO-A1-2004/022020
- JP-A- 2006 169 129

## Beschreibung

Die vorliegende Erfindung betrifft eine kosmetische Zubereitung enthaltend 2-(4'-Diethylamino-2'-hydoxybenzoyl)-benzoesäurehexylester, Diole und Ölsäure.

Der Wunsch, schön und attraktiv auszusehen, ist von Natur aus im Menschen verwurzelt. Auch wenn das Schönheitsideal im Laufe der Zeit Wandlungen erfahren hat, so ist das Streben nach einem makellosen Äußeren immer das Ziel der Menschen gewesen. Einen wesentlichen Anteil an einem schönen und attraktiven Äußeren hat dabei der Zustand und das Aussehen der Haut.

Damit die Haut ihre biologischen Funktionen im vollen Umfang erfüllen kann, bedarf sie der regelmäßigen Reinigung und Pflege. Die Reinigung der Haut dient dabei der Entfernung von Schmutz, Schweiß und Resten abgestorbener Hautpartikel, die einen idealen Nährboden für Krankheitserreger und Parasiten aller Art bilden. Hautpflegeprodukte dienen meist der Befeuchtung und Rückfettung der Haut. Häufig sind ihnen Wirkstoffe zugesetzt, welche die Haut regenerieren oder vor den schädlichen Einflüssen der UV-Strahlung schützen.

Eine besondere Produktform von Hautpflegeprodukten stellen die Tagespflegeprodukte dar. Tagespflegeprodukte unterscheiden sich von den so genannten Nachtpflegeprodukten insbesondere darin, dass sie UV-Lichtschutzfilter enthalten (die man nachts nicht benötigt), weniger stark fettend sind und schneller in die Haut einziehen. Tagespflegeprodukte können darüber hinaus mit Farbstoffen und Farbpigmenten versetzt sein, die der Haut nach dem Auftragen ein gesundes Aussehen verleihen. In der Regel enthalten sie Hautbefeuchtungsmittel (Moisturizer wie Glycerin oder Diole), kosmetische Wirkstoffe (beispielsweise gegen Falten) und andere Pflegestoffe (beispielsweise Ölkomponenten zur Rückfettung der Haut).

Nachteilig am Stande der Technik ist der Umstand, dass eine Reihe von Inhaltsstoffen von Tagespflegeprodukten beispielsweise aufgrund ihres Reinheitsgrades, ihrer Oxidationsempfindlichkeit oder schlicht ihrer Stoffeigenschaften, einen gewissen Eigengeruch aufweisen, der in der Regel als unangenehm und "unkosmetisch" empfunden wird. Das Problem des unerwünschten Eigengeruches tritt beispielsweise bei Diolen und Ölsäure auf.

Derartige Eigengerüche von Inhaltsstoffen beeinträchtigen darüber hinaus das Duftbouquet der Parfümkomposition. Kurz: Derartige Eigengerüche von Inhaltsstoffen sind unerwünscht und müssen in der Regel mit einem übermäßigen Einsatz von teuren (und bei Einzelkomponenten manchmal auch dermatologisch nicht ganz unbedenklichen) Parfümstoffen überdeckt werden.

Ein wesentlicher Bestandteil moderner Tagespflegeprodukte sind UV-A-Lichtschutzfilter, welche die Haut vor den schädlichen Einflüssen der UV-A-Strahlung schützen sollen. Derartige UV-A-Filter wie beispielsweise 2-(4'-Diethylamino-2'-hydoxybenzoyl)-benzoesäurehexylester haben den Nachteil, das sie nicht ganz einfach in die Zubereitung einzuarbeiten sind, insbesondere dann, wenn vermieden werden soll, dass dieser intensiv gelb färbende Filter die Zubereitung allzu intensiv gelb färbt.

Es war daher die Aufgabe der vorliegenden Erfindung, die Mängel des Standes der Technik zu beseitigen. Insbesondere war es die Aufgabe der vorliegenden Erfindung kosmetische Zubereitungen, insbesondere Tagespflegezubereitungen zu entwickeln, bei denen der Eigengeruch von Inhaltsstoffen, insbesondere von Diolen und Ölsäure, wirkungsvoll unterdrückt wird.

Überraschend gelöst wird die Aufgabe durch eine kosmetische Zubereitung gemäß Anspruch 1.

Überraschend gelöst wird die Aufgabe ferner durch die Verwendung von 2-(4'-Diethylamino-2'-hydoxybenzoyl)-benzoesäurehexylester zur Unterdrückung des Eigengeruches von Diolen und Ölsäure in kosmetischen Zubereitungen.

Im Rahmen dieser Offenbarung beziehen sich die Formulierungen "erfindungsgemäß vorteilhaft", "erfindungsgemäß bevorzugt" etc. sowohl auf die erfindungsgemäße Zubereitung als auch auf die erfindungsgemäße Verwendung.

Die erfindungsgemäßen Zubereitungen lassen sich einfach und kostengünstig herstellen.

Zwar kennt der Stand der Technik dieWO 2004/022020 sowie die JP2006-169129, doch konnten diese Schriften nicht den Weg zur vorliegenden Erfindung weisen.

Es ist erfindungsgemäß vorteilhaft, wenn ein oder mehrere Diole gewählt werden aus der Gruppe der Verbindungen 2-Methyl-1,3-propandiol, Pentan-1,2-diol, Hexan-1,2-diol, Heptan-1,2-diol, Octan-1,2-diol, Nonan-1,2-diol, Decan-1,2-diol.

Erfindungsgemäß bevorzugt sind dabei die Diole Octan-1,2-diol, Hexan-1,2-diol.

Es ist erfindungsgemäß vorteilhaft, wenn die Zubereitung ein oder mehrere Diole in einer Gesamtkonzentration von 0,1 bis 5 Gewichts- %, bezogen auf das Gesamtgewicht der Zubereitung, enthält.

Es ist erfindungsgemäß bevorzugt, wenn die Zubereitung ein oder mehrere Diole in einer Gesamtkonzentration von 0,1 bis 3 Gewichts- %, bezogen auf das Gesamtgewicht der Zubereitung, enthält.

Es ist erfindungsgemäß vorteilhaft, wenn die Zubereitung 2-(4'-Diethylamino-2'-hydoxybenzoyl)-benzoesäurehexylester in einer Konzentration von 0,1 bis 10 Gewichts-%, bezogen auf das Gesamtgewicht der Zubereitung, enthält.

Es ist erfindungsgemäß bevorzugt, wenn die Zubereitung 2-(4'-Diethylamino-2'-hydoxybenzoyl)-benzoesäurehexylester in einer Konzentration von 0,1 bis 6 Gewichts-%, bezogen auf das Gesamtgewicht der Zubereitung, enthält.

Erfindungsgemäß vorteilhafte Ausführungsformen der vorliegenden Erfindung sind dadurch gekennzeichnet, dass die Zubereitung einen oder mehrere Farbstoffe enthält. Erfindungsgemäß vorteilhaft, wenn die Zubereitung einen oder mehrere Farbstoffe in einer Gesamtkonzentration von 0,001 bis 1,0 Gewichts-%, bezogen auf das Gesamtgewicht der Zubereitung, enthält.

Erfindungsgemäß bevorzugt, wenn die Zubereitung eine oder mehrere Farbstoffe in einer Gesamtkonzentration von 0,01 bis 0,5 Gewichts-%, bezogen auf das Gesamtgewicht der Zubereitung, enthält.

Enthalten die erfindungsgemäßen Zubereitungen Farbstoffe, ist es erfindungsgemäß vorteilhaft, wenn die Zubereitung einen oder mehrere Farbstoffe gewählt aus der Gruppe der Farbstoffe mit dem Color-Index CI 16035 (Allurarot, 6-Hydroxy-5-(2-methoxy-5-methyl-4-sulfophenylazo)-2-naphthalinsulfonsäuredinatriumsalz) und CI 15985 (1-(4-Sulfo-1-phenylazo)-2-naphthol-6-sulfosäure) enthält.

Erfindungsgemäß vorteilhaft, wenn die Zubereitung CI 16035 in einer Gesamtkonzentration von 0,01 bis 1 Gewichts- %, bezogen auf das Gesamtgewicht der Zubereitung, enthält.

Erfindungsgemäß bevorzugt, wenn die Zubereitung CI 16035 in einer Gesamtkonzentration von 0,01 bis 0,5 Gewichts- %, bezogen auf das Gesamtgewicht der Zubereitung, enthält.

Erfindungsgemäß vorteilhaft, wenn die Zubereitung CI 15985 in einer Gesamtkonzentration von 0,01 bis 1 Gewichts-%, bezogen auf das Gesamtgewicht der Zubereitung, enthält.

Erfindungsgemäß bevorzugt, wenn die Zubereitung CI 15985 in einer Gesamtkonzentration von 0,01 bis 0,5 Gewichts-%, bezogen auf das Gesamtgewicht der Zubereitung, enthält.

Es ist erfindungsgemäß von Vorteil, wenn die erfindungsgemäße Zubereitung Farbpigmente enthält. Farbpigmente werden erfindungsgemäß von den Farbstoffen dadurch unterschieden, dass es sich bei den Pigmenten um ungelöste farbgebende Mittel handelt, die in pimentärer Form in der Zubereitung vorliegen. Erfindungsgemäß bevorzugt ist dabei der Einsatz anorganischer Farbpigmente, insbesondere Titandioxid, Eisenoxide und/oder Bariumsulfat.

Erfindungsgemäß vorteilhaft, wenn die Zubereitung Titandioxid als Weißpigment in einer Gesamtkonzentration von 0,5 bis 30 Gewichts-%, bezogen auf das Gesamtgewicht der Zubereitung, enthält.

Erfindungsgemäß bevorzugt, wenn die Zubereitung Titandioxid als Weißpigment in einer Gesamtkonzentration von 0,5 bis 15 Gewichts-%, bezogen auf das Gesamtgewicht der Zubereitung, enthält.

Erfindungsgemäß vorteilhaft, wenn die Zubereitung Eisenoxide in einer Gesamtkonzentration von 0,1 bis 10 Gewichts-%, bezogen auf das Gesamtgewicht der Zubereitung, enthält.

Erfindungsgemäß bevorzugt, wenn die Zubereitung Eisenoxide in einer Gesamtkonzentration von 0,5 bis 5 Gewichts-%, bezogen auf das Gesamtgewicht der Zubereitung, enthält.

Es ist erfindungsgemäß vorteilhaft, wenn die Zubereitung Ölsäure in einer Konzentration von 0,1 bis 10 Gewichts-%, bezogen auf das Gesamtgewicht der Zubereitung, enthält.

Es ist erfindungsgemäß bevorzugt, wenn die Zubereitung Ölsäure in einer Konzentration von 0,1 bis 5 Gewichts-%, bezogen auf das Gesamtgewicht der Zubereitung, enthält.

Obwohl die erfindungsgemäße Zubereitung als Oleogel vorliegen kann, ist es erfindungsgemäß vorteilhaft, wenn die Zubereitung in Form einer Emulsion oder Dispersion vorliegt. Erfindungsgemäß besonders bevorzugt sind dabei O/W-Emulsionen.

Liegt die erfindungsgemäße Zubereitung in Form einer OW-Emulsion vor, so ist sie erfindungsgemäß bevorzugt dadurch gekennzeichnet, dass die Zubereitung einen oder mehrere O/W-Emulgatoren gewählt aus der Gruppe der Verbindungen Glycerylstearatcitrat, Glycerylstearat (selbstemulgierend), Stearinsäure, Stearatsalze, Polyglyceryl-3-methylglycosedistearat, Ceteareth-20, PEG-40 Stearat, Natriumcetearylsulfat enthält. Ferner ist es vorteilhaft im Sinne der vorliegenden Erfindung Cetearylalkohol in Kombination mit PEG-40 hydriertes Rizinusöl, Natriumcetearylsulfat und Glycerylstearat Sucrose Polystearat, Natrium Stearoyl Glutamat. Außerdem ist es erfindungsgemäß vorteilhaft Kaliumcetylphosphat als Emulgator einzusetzen.

Diese erfindungsgemäßen O/W-Emulgatoren können erfindungsgemäß vorteilhaft in einer Konzentration von 0,001 bis 10 Gewichts-% und bevorzugt in einer Konzentration von 0,1 bis 7 Gewichts-%, bezogen auf das Gesamtgewicht der Zubereitung in dieser enthalten sein.

In einer weiteren erfindungsgemäßen Ausführungsform liegt die erfindungsgemäße Zubereitung in Form einer W/O-Emulsion vor.

In dieser Ausführungsform ist es erfindungsgemäß bevorzugt, wenn die Zubereitung einen oder mehrere W/O-Emulgatoren gewählt aus der Gruppe der Verbindungen Polyglyceryl-2-dipolyhydroxystearat, PEG-30 Dipolyhydroxystearat, Cetyl Dimethicon Copolyol, Polyglyceryl-3 Diisostearat enthält.

Diese erfindungsgemäßen W/O-Emulgatoren können erfindungsgemäß vorteilhaft in einer Konzentration von 0,1 bis 10 Gewichts-% und bevorzugt in einer Konzentration von 0,2 bis 7 Gewichts-%, bezogen auf das Gesamtgewicht der Zubereitung in dieser enthalten sein.

Eine erfindungsgemäß vorteilhafte Ausführungsform ist dadurch gekennzeichnet, dass die Zubereitung einen oder mehrere weitere UV-Filter enthält, gewählt aus der Gruppe der Verbindungen Phenylen-1,4-bis-(2-benzimidazyl)-3,3'-5,5'-tetrasulfonsäuresalze; 2-Phenylbenzimidazol-5-sulfonsäuresalze; 1,4-di(2-oxo-10-Sulfo-3-bornylidenmethyl)-Benzol und dessen Salze; 4-(2-Oxo-3-bomylidenmethyl)benzolsulfonsäuresalze; 2-Methyl-5-(2-oxo-3-bornylidenmethyl)sulfonsäuresalze; 2,2'-Methylen-bis-(6-(2H-benzotriazol-2-yl)-4-(1,1,3,3-tetramethylbutyl)-phenol); 2-(2H-benzotriazol-2-yl)-4-methyl-6-[2-methyl-3-[1,3,3,3-tetramethyl-1-[(trimethylsilyl)oxy]disiloxanyl]propyl]-phenol; 3-(4-Methylbenzyliden)campher; 3-Benzylidencampher; Ethylhexylsalicylat; Terephthalidendicamphersulfonsäure; 4-(Dimethylamino)-benzoesäure(2-ethylhexyl)ester; 4-(Dimethylamino)benzoesäure-amylester; 4-Methoxybenzalmalon-säuredi(2-ethylhexyl)ester; 4-Methoxyzimtsäure(2-ethylhexyl)ester; 4-Methoxyzimtsäureisoamylester; 2-Hydroxy-4-methoxybenzophenon, 2-Hydroxy-4-methoxy-4'-methylbenzophenon; 2,2'-Dihydroxy-4-methoxybenzophenon; 4-(tert.-Butyl)-4'-methoxydibenzoylmethan; Homomenthylsalicylat; 2-Ethylhexyl-2-hydroxybenzoat; 2-Ethylhexyl-2-cyano-3,3-diphenylacrylat; Dimethicodiethylbenzalmalonat; 3-(4-(2,2-bis Ethoxycarbonylvinyl)-phenoxy)propenyl)-methoxysiloxan / Dimethylsiloxan - Copolymer; Dioctylbutylamidotriazon (INCI: Diethylhexyl-Butamidotriazone); 2,4-bis-[5-1(dimethylpropyl)benzoxazol-2-yl-(4-phenyl)-imino]-6-(2-ethylhexyl)-imino-1,3,5-triazin mit der (CAS Nr. 288254-16-0); 4,4',4"-(1,3,5-Triazin-2,4,6-triyltriimino)-tris-benzoesäure-tris(2-ethylhexyl-ester) (auch: 2,4,6-Tris-[anilino-(p-carbo-2'-ethyl-1'-hexyloxy)]-1,3,5-triazin (INCI: Ethylhexyl Triazone); 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazin); 2,4,6-Tris-(biphenyl)-1,3,5-triazin; 2,4-Bis-(4'-Di-neopentylaminobenzalmalonat)-6-(4"-butylaminobenzoat)-s-triazin, Titandioxid, Zinkoxid, Merocyanine gewählt aus der Gruppe der Verbindungen

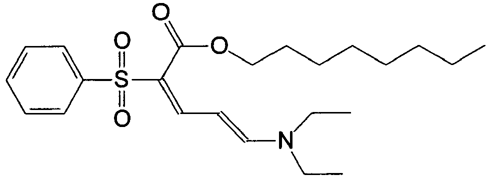

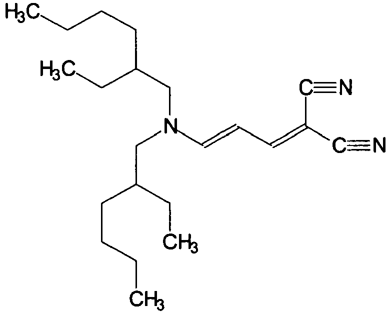

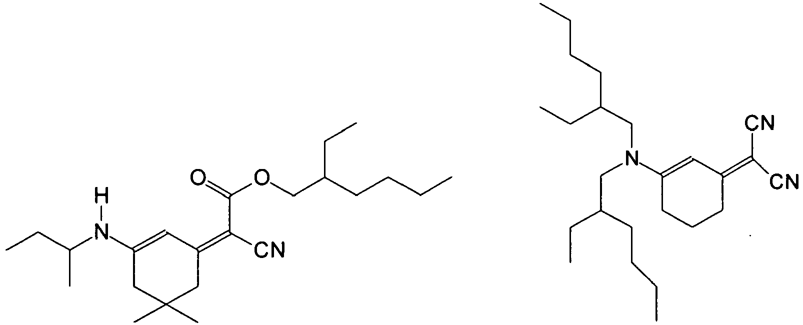

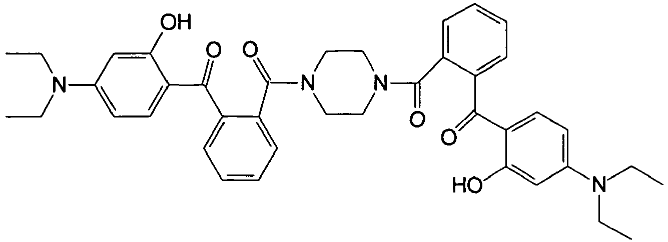

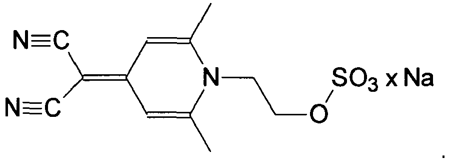

Es ist erfindungsgemäß vorteilhaft, wenn die erfindungsgemäße Zubereitung frei ist von p-Methylbenzylidencampher.

Es ist dabei erfindungsgemäß vorteilhaft, wenn die erfindungsgemäße Zubereitung einen oder mehrere dieser zusätzlichen UV-Filter in einer Gesamtkonzentration von 0,1 bis 40 Gewichts-% und bevorzugt in einer Konzentration von 1 bis 30 Gewichts-%, bezogen auf das Gesamtgewicht der Zubereitung, enthält.

Eine alternative erfindungsgemäße Ausführungsform der vorliegenden Erfindung ist dadurch gekennzeichnet, dass die Zubereitung keine UV-B-Filter enthält.

Es ist erfindungsgemäß vorteilhaft, wenn die Zubereitung als weitere Inhaltsstoffe eine oder mehrere Verbindungen gewählt aus der Gruppe der Verbindungen alpha-Liponsäure, Folsäure, Phytoen, D-Biotin, Coenzym Q10, Niacinamid, alpha-Glucosylrutin, Carnitin, Carnosin, natürliche und/oder synthetische Isoflavonoide, Flavonoide, Kreatin, Kreatinin, Taurin, β-Alanin, Tocopherylacetat, Dihydroxyaceton; 8-Hexadecen-1,16-dicarbonsäure, Glycerylglycose, (2-Hydroxyethyl)harnstoff, Vitamin E bzw. seine Derivate und/oder Licochalcon A in den üblichen Einsatzkonzentrationen enthält.

Es ist erfindungsgemäß vorteilhaft, wenn die Zubereitung ein oder mehrere Parabene (z.B. Methylparaben, Ethylparaben, Propylparaben, Butylparaben) enthält.

Dabei ist ein Gesamt-Paraben-Gehalt von 0,05 bis 1 Gewichts-%, bezogen auf das Gesamtgewicht der Zubereitung erfindungsgemäß vorteilhaft.

Die erfindungsgemäßen Zubereitungen können ferner vorteilhaft auch Selbstbräunungssubstanzen enthalten, wie beispielsweise Dihydroxyaceton und/oder Melaninderivate in Konzentrationen von 1 Gew.-% bis zu 10 Gew.-%, bezogen auf das Gesamtgewicht der Zubereitung.

Ferner können die erfindungsgemäßen Zubereitungen auch Repellentien zum Schutz vor Mücken, Zecken und Spinnen und dergleichen enthalten. Vorteilhaft sind z. B. N,N-Diethyl-3-methylbenzamid (Handelsbezeichnung: Meta-delphene, "DEET"), Dimethylphtalat (Handelsbezeichnung: Palatinol M, DMP), 1-Piperidincarbonsäure-2-(2-hydroxyethyl)-1-methylpropylester sowie insbesondere 3-(N-n-Butyl-N-acetyl-amino)-propionsäureethylester (unter dem Handelsnamen Insekt Repellent® 3535 bei der Fa. Merck erhältlich). Die Repellentien können sowohl einzeln als auch in Kombination eingesetzt werden.

Als Feuchthaltemittel (Moisturizer) werden Stoffe oder Stoffgemische bezeichnet, welche kosmetischen Zubereitungen die Eigenschaft verleihen, nach dem Auftragen bzw. Verteilen auf der Hautoberfläche die Feuchtigkeitsabgabe der Hornschicht (auch transepidermal water loss (TEWL) genannt) zu reduzieren und/oder die Hydratation der Hornschicht positiv zu beeinflussen.

Weitere erfindungsgemäß vorteilhafte Feuchthaltemittel (Moisturizer) im Sinne der vorliegenden Erfindung (neben den Diolen) sind beispielsweise Glycerin, Milchsäure und/oder Lactate, insbesondere Natriumlactat, Butylenglykol, Propylenglykol, Biosaccaride Gum-1, Glycine Soja, Ethylhexyloxyglycerin, Pyrrolidoncarbonsäure und Harnstoff. Ferner ist es insbesondere von Vorteil, polymere Moisturizer aus der Gruppe der wasserlöslichen und/oder in Wasser quellbaren und/oder mit Hilfe von Wasser gelierbaren Polysaccharide zu verwenden. Insbesondere vorteilhaft sind beispielsweise Hyaluronsäure, Chitosan und/oder ein fucosereiches Polysaccharid, welches in den Chemical Abstracts unter der Registraturnummer 178463-23-5 abgelegt und z. B. unter der Bezeichnung Fucogel®1000 von der Gesellschaft SOLABIA S.A. erhältlich ist. Moisturizer können vorteilhaft auch als Antifaltenwirkstoffe zum Schutz vor Hautveränderungen, wie sie z. B. bei der Hautalterung auftreten, verwendet werden.

Es ist vorteilhaft im Sinne der vorliegenden Erfindung, wenn die erfindungsgemäße Zubereitung ein oder mehrere der weiteren Feuchthaltemittel in einer Gesamtkonzentration von 0,1 bis 20 Gewichts-% und bevorzugt in einer Gesamtkonzentration von 0,5 bis 10 Gewichts-%, jeweils bezogen auf das Gesamtgewicht der Zubereitung, enthält.

Die erfindungsgemäßen kosmetischen Zubereitungen können ferner vorteilhaft, wenngleich nicht zwingend, Füllstoffe enthalten, welche z. B. die sensorischen und kosmetischen Eigenschaften der Formulierungen weiter verbessern und beispielsweise ein samtiges oder seidiges Hautgefühl hervorrufen oder verstärken. Vorteilhafte Füllstoffe im Sinne der vorliegenden Erfindung sind Stärke und Stärkederivate (wie z. B. Tapiocastärke, Distärkephosphat, Aluminium- bzw. Natrium-Stärke Octenylsuccinat und dergleichen), Pigmente, die weder hauptsächlich UV-Filter- noch färbende Wirkung haben (wie z. B. Bornitrid etc.) und/oder Aerosile® (CAS-Nr. 7631-86-9) und /oder Talkum. Erfindungsgemäß vorteilhaft enthält die erfindungsgemäße Zubereitung Filmbildner. Filmbildner im Sinne der vorliegenden Erfindung sind Stoffe unterschiedlicher Zusammensetzung, die durch die folgende Eigenschaft charakterisiert sind: Löst man einen Filmbildner in Wasser oder anderen geeigneten Lösungsmitteln und trägt die Lösung dann auf die Haut auf, so bildet er nach dem Verdunsten des Lösemittels einen Film aus, der im wesentlichen dazu dient, die Lichtfilter auf der Haut zu fixieren und so die Wasserfestigkeit des Produktes zu steigern.

Es ist insbesondere von Vorteil, die Filmbildner aus der Gruppe der Polymere auf Basis von Polyvinylpyrrolidon (PVP) 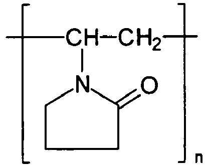 zu wählen. Besonders bevorzugt sind Copolymere des Polyvinylpyrrolidons, beispielsweise das PVP Hexadecen Copolymer und das PVP Eicosen Copolymer, welche unter den Handelsbezeichnungen Antaron V216 und Antaron V220 bei der GAF Chemicals Cooperation erhältlich sind.

Ebenfalls vorteilhaft sind weitere polymere Filmbildner, wie beispielsweise Natriumpolystryrensulfonat, welches unter der Handelsbezeichnung Flexan 130 bei der National Starch and Chemical Corp. erhältlich ist, und/oder Polyisobuten, erhältlich bei Rewo unter der Handelsbezeichnung Rewopal PIB1000. Weitere geeignete Polymere sind z.B. Polyacrylamide (Seppigel 305), Polyvinylalkohole, PVP, PVP / VA Copolymere, Polyglycole, Acrylat/Octylacralymid Copolymer (Dermacryl 79). Ebenfalls vorteilhaft ist die Verwendung von Hydriertem Rizinusöl Dimerdilinoleat (CAS 646054-62-8, INCI Hydrogenated Castor Oil Dimer Dilinoleate), das bei der Firma Kokyu Alcohol Kogyo unter dem Namen Risocast DA-H erworben werden kann oder aber auch PPG-3 Benzylethermyristat (CAS 403517-45-3), das unter dem Handelsnamen Crodamol STS bei der Firma Croda Chemicals erworben werden kann.

Die Ölphase der erfindungsgemäßen Zubereitung wird vorteilhaft gewählt aus der Gruppe der polaren Öle, beispielsweise aus der Gruppe der Lecithine und der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, wie z. B. Cocoglycerid, Olivenöl, Sonnenblumenöl, Jojobaöl, Sojaöl, Erdnußöl, Rapsöl, Mandelöl, Palmöl, Kokosöl, Rizinusöl, Weizenkeimöl, Traubenkernöl, Distelöl, Nachtkerzenöl, Macadamianußöl und dergleichen mehr. sowie Propylheptyl Octanoat und/oder Diisopropylsebacat. Avocadoöl und Babassuöl, Mandelöl

Erfindungsgemäß vorteilhaft sind ferner z. B. natürliche Wachse tierischen und pflanzlichen Ursprungs, wie beispielsweise Bienenwachs und andere Insektenwachse sowie Beerenwachs, Sheabutter und/oder Lanolin (Wollwachs).

Weitere vorteilhafte polare Ölkomponenten können im Sinne der vorliegenden Erfindung ferner gewählt werden aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen sowie aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Phenethylbenzoat, 2-Phenylethylbenzoat, Isopropyl Lauroyl Sarkosinat, Phenyl Trimethicon, Cyclomethicon, Dibutyladipat, Octylpalmitat, Octylcocoat, Octylisostearat, Isopropyl palmitat, Isostearyl Isostearat, Cetearyl Isononanoate, Isopropyl Stearat Octyldodeceylmyristat, Octyldodekanol, Cetearylisononanoat, Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Stearylheptanoat, Oleyloleat, Oleylerucat, Erucyloleat, Erucylerucat, Tridecylstearat, Tridecyltrimellitat, sowie synthetische, halbsynthetische und natürliche Gemische solcher Ester, wie z. B. Jojobaöl.

Ferner kann die Ölphase vorteilhaft gewählt werden aus der Gruppe der Dialkylether und Dialkylcarbonate, vorteilhaft sind z. B. Dicaprylylether (*Cetiol OE*) und/oder Dicaprylylcarbonat, beispielsweise das unter der Handelsbezeichnung *Cetiol CC* bei der Fa. Cognis erhältliche.

Es ist ferner bevorzugt, das oder die Ölkomponenten aus der Gruppe Isoeikosan, Neopentylglykoldiheptanoat, Propylenglykoldicaprylat/dicaprat, Caprylic/Capric/Diglycerylsuccinat, Butylenglykol Dicaprylat/Dicaprat, C₁₂₋₁₃-Alkyllactat, Di-C₁₂₋₁₃-Alkyltartrat, Triisostearin, Dipentaerythrityl Hexacaprylat/Hexacaprat, Propylenglykolmonoisostearat, Tricaprylin, Dimethylisosorbid. Es ist insbesondere vorteilhaft, wenn die Ölphase der erfindungsgemäßen Formulierungen einen Gehalt an C₁₂₋₁₅-Alkylbenzoat aufweist oder vollständig aus diesem besteht.

Vorteilhafte Ölkomponenten sind ferner z. B. Butyloctylsalicylat (beispielsweise das unter der Handelsbezeichnung *Hallbrite BHB* bei der Fa. CP Hall erhältliche), Tridecylsalicylat (welches unter der Handelsbezeichnung Cosmacol ESI bei der Fa. Sasol erhältlich ist), C12-C15 Alkylsalicylat (unter der Handelsbezeichnung Dermol NS bei der Fa. Alzo erhältlich), Hexadecylbenzoat und Butyloctylbenzoat und Gemische davon (*Hallstar AB*) und/oder Diethylhexylnaphthalat (*Hallbrite TQ oder Corapan TQ von Symrise*).

Auch beliebige Abmischungen solcher Öl- und Wachskomponenten sind vorteilhaft im Sinne der vorliegenden Erfindung einzusetzen.

Ferner kann die Ölphase ebenfalls vorteilhaft auch unpolare Öle enthalten, beispielsweise solche, welche gewählt werden aus der Gruppe der verzweigten und unverzweigten Kohlenwasserstoffe und -wachse, insbesondere Mineralöl, Vaseline (Petrolatum), Paraffinöl, Squalan und Squalen, Polyolefine, hydrogenierte Polyisobutene, C13-16 Isoparaffin und Isohexadecan. Unter den Polyolefinen sind Polydecene die bevorzugten Substanzen.

Die erfindungsgemäßen Zubereitungen können ferner vorteilhaft eine oder mehrere Substanzen aus der folgenden Gruppe der Siloxanelastomere enthalten, beispielsweise um die Wasserfestigkeit und/oder den Lichtschutzfaktor der Produkte zu steigern:
(a) Siloxanelastomere, welche die Einheiten R₂SiO und RSiO_{1,5} und/oder R₃SiO_{0,5} und/oder SiO₂ enthalten,
   wobei die einzelnen Reste R jeweils unabhängig voneinander Wasserstoff, C₁₋₂₄-Alkyl (wie beispielsweise Methyl, Ethyl, Propyl) oder Aryl (wie beispielsweise Phenyl oder Tolyl), Alkenyl (wie beispielsweise Vinyl) bedeuten und das Gewichtsverhältnis der Einheiten R₂SiO zu RSiO_{1,5} aus dem Bereich von 1 : 1 bis 30 : 1 gewählt wird;
(b) Siloxanelastomere, welche in Silikonöl unlöslich und quellfähig sind, die durch die Additionsreaktion eines Organopolysiloxans (1), das siliciumbebundenen Wasserstoff enthält, mit einem Organopolysiloxan (2), das ungesättigte aliphatische Gruppen enthält, erhältlich sind,
   wobei die verwendeten Mengenateile so gewählt werden, dass die Menge des Wasserstoffes des Organopolysiloxans (1) oder der ungesättigten aliphatischen Gruppen des Organopolysiloxans (2)
   - im Bereich von 1 bis 20 mol-% liegt, wenn das Organopolysiloxan nicht zyklisch ist und
   - im Bereich von 1 bis 50 mol-% liegt, wenn das Organopolysiloxan zyklisch ist.

Vorteilhaft im Sinne der vorliegenden Erfindung liegen das oder die Siloxanelastomere in Form sphärischer Puder oder in Form von Gelen vor.

Erfindungsgemäß vorteilhafte in Form sphärischer Puder vorliegende Siloxanelastomere sind die mit der INCI-Bezeichnung Dimethicone / Vinyl Dimethicone Crosspolymer, beispielsweise das von DOW CORNING unter der Handelsbezeichnungen DOW CORNING 9506 Powder erhältliche.

Besonders bevorzugt ist es, wenn das Siloxanelastomer in Kombination mit Ölen aus Kohlenwasserstoffen tierischer und/oder pflanzlicher Herkunft, synthetischen Ölen, synthetischen Estern, synthetischen Ethern oder deren Gemischen verwendet wird.

Besonders vorteilhafte Zubereitungen werden ferner erhalten, wenn als Zusatz- oder Wirkstoffe Antioxidantien eingesetzt werden. Erfindungsgemäß enthalten die Zubereitungen vorteilhaft eines oder mehrere Antioxidantien. Als günstige, aber dennoch fakultativ zu verwendende Antioxidantien können alle für kosmetische Anwendungen geeigneten oder gebräuchlichen Antioxidantien verwendet werden.

Besonders vorteilhaft im Sinne der vorliegenden Erfindung können wasserlösliche Antioxidantien eingesetzt werden, wie beispielsweise Vitamine, z. B. Ascorbinsäure und deren Derivate.

Bevorzugte Antioxidantien sind ferner Vitamin E und dessen Derivate sowie Vitamin A und dessen Derivate.

Die Menge der Antioxidantien (eine oder mehrere Verbindungen) in den Zubereitungen beträgt vorzugsweise 0,001 bis 30 Gew.-%, besonders bevorzugt 0,05 bis 20 Gew.-%, insbesondere 0,1 bis 10 Gew.-%, bezogen auf das Gesamtgewicht der Zubereitung.

Die kosmetischen Zubereitungen gemäß der Erfindung können kosmetische Hilfsstoffe enthalten, wie sie üblicherweise in solchen Zubereitungen verwendet werden.

Vorteilhaft im Sinne der vorliegenden Erfindung sind Zubereitungen zur Pflege der Haut: sie können dem kosmetischen Lichtschutz, ferner als Schminkprodukt in der dekorativen Kosmetik dienen.

Erfindungsgemäß ist insbesondere die Verwendung der erfindungsgemäßen Zubereitung zum Schutz vor Hautalterung (insbesondere zum Schutz vor UV-bedingter Hautalterung) sowie als Sonnenschutzmittel.

Erfindungsgemäß vorteilhaft weist die erfindungsgemäße Zubereitung einen pH-Wert von 5 bis 8 auf. Dieser kann durch die herkömmlichen Säuren, Basen und Puffersysteme eingestellt werden.

Erfindungsgemäß ist die Verwendung der Zubereitung in kosmetischen Sprays (insbesondere Sonnenschutzmitteln) oder als Tränkmedium für Pads und Tücher.

### Beispiele

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

**O/W Emulsion**

| | **1** | **2** | **3** | **4** | **5** | **6** |
|---|---|---|---|---|---|---|
| Glyceryl Stearat Citrat | 2 | 2 | 3 | | | |
| Glyceryl Stearat SE | | | | 1 | 1 | 1,5 |
| Cetearyl Alkohol + PEG-40 Rizinusöl+ Natrium Cetearyl Sulfat | | | | 2,5 | 2,5 | 3 |
| Cetearylalkohol | | | 1 | 1 | | |
| Stearylalkohol | 0,5 | | | | | 2 |
| Myristylmyristat | 1,0 | 1 | | | 3 | |
| Acrylates/C₁₀₋₃₀ Alkyl Acrylat Crosspolymer | 0,1 | 0,2 | | | 0,1 | |
| Carbomer | | 0,2 | 0,3 | 0,2 | | |
| Xanthan Gum | 0,4 | | 0,2 | 0,2 | 0,3 | 0,4 |
| C₁₂₋₁₅ Alkyl Benzoat | | 3 | | | 5 | |
| Butylenglycol Dicaprylat/Dicaprat | 5 | | | | 3 | 3 |
| Methylpropandiol | | 1 | | 0,5 | | |
| Dicaprylcaprat | 2 | 2 | | | 2 | 2 |
| 1,2-Hexandiol | 0,2 | | 0,1 | 0,3 | 0,1 | 0,1 |
| 1,2-Octandiol | | 0,2 | 0,1 | | 0,3 | |
| Cyclomethicon | | | 5 | 10 | | |
| PVP Hexadecen Copolymer | | 0,5 | | | | 1 |
| Propylenglykol | | | 1 | | 5 | 3 |
| Glycerin | 7,5 | 5 | 7 | 10 | 13 | 3 |
| Alkohol denat. | 2 | 3 | | 7 | | |
| Titandioxid | 3 | | | 2 | | |
| Ethylhexyltriazin | 2,5 | 2 | | 1 | | |
| 4-Methoxyzimtsäure(2-ethylhexyl)ester | | 5 | | 2 | | |
| Octocrylen | 7,5 | 8 | 3 | 2 | 2 | 1 |
| Butyl Methoxydibenzoylmethan | 3,8 | 4 | 3,5 | 2 | | |
| Phenylbenzimidazol Sulfonsäure | | | | 2,5 | 3 | 2 |
| 2-(4'-Diethylamino-2'-hydoxybenzoyl)-benzoesäurehexylester | 1 | 2 | 5 | 3 | 7,5 | 10 |
| 2,2'-Methylen-bis-(6-(2H-benzotriazol-2-yl)-4-(1,1,3.3-tetramethylbutyl)-phenol) | | 4 | | | 2 | |
| 2,4,6-Tris-(biphenyl)-1,3,5-triazin | | | 1 | 3 | | |
| Ölsäure | 0,5 | 0,8 | 0,8 | 1 | 0,2 | 1,8 |
| Ethylhexylsalicylat | 2 | | 0,5 | 4 | 9,5 | |
| Bis-Ethylhexyloxyphenol Methoxyphenyltriazin | | 1 | | 1 | | 1 |
| Vitamin E Acetat | 0,2 | 0,2 | 0,2 | 0,3 | 0,1 | 0,5 |
| Na₂H₂EDTA | 0,1 | 0,1 | 0,2 | 0,2 | 0,2 | 0,5 |
| Parfüm, Konservierungsmittel | q.s. | q.s. | q.s. | q.s. | q.s. | q.s. |
| Farbstoffe, usw. | q.s. | q.s. | q.s. | q.s. | q.s. | q.s. |
| Citronensäure, Natriumcitrat | q.s. | q.s. | q.s. | q.s. | q.s. | q.s. |
| Natriumhydroxid | q.s. | q.s. | q.s. | q.s. | q.s. | q.s. |
| Wasser | ad 100,0 | ad 100,0 | ad 100,0 | ad 100,0 | ad 100,0 | ad 100,0 |

| | **7** | **8** | **9** | **10** | **11** | **12** |
|---|---|---|---|---|---|---|
| Glyceryl Stearat Citrat | 2 | 2 | 3 | | | |
| Glyceryl Stearat SE | | | | 1 | 1 | 1,5 |
| Cetearyl Alkohol | | | | 1,9 | 2,0 | 1,35 |
| PEG-40 Castoröl | | | | 0,375 | 0,4 | 0,225 |
| Natrium Cetearyl Sulfat | | | | 0,19 | 0,18 | 0,11 |
| Cetearylalkohol | | | 1 | 1 | | |
| Stearylalkohol | 0,5 | | | | | 2 |
| Myristyl myristat | 1,0 | 1 | | | 3 | 2 |
| Acrylat/C₁₀₋₃₀ Alkyl Acrylat Crosspolymer | 0,1 | 0,2 | | | 0,1 | |
| Carbomer | | 0,2 | 0,3 | 0,2 | | |
| Xanthan Gum | 0,4 | | 0,2 | 0,2 | 0,3 | 0,4 |
| C₁₂₋₁₅ Alkyl Benzoat | | 3 | | | 5 | |
| Butylenglycol Dicaprylat/Dicaprat | 5 | | | | 3 | 3 |
| Dicaprylcaprat | 2 | 2 | | | 2 | 2 |
| Cyclomethicon | | | 5 | 10 | | |
| Dimethicon | | | | 5 | | |
| PVP Hexadecen Copolymer | | 0,5 | | | | 1 |
| Propylenglykol | | | 1 | | 5 | 3 |
| Glycerin | 7,5 | 5 | 7 | 10 | 13 | 3 |
| Alkohol denat. | 2 | 3 | | 7 | | |
| Titandioxid | 3 | | | 2 | | |
| Merocyanin | 2 | | 2 | | 3 | 3 |
| Ethylhexyltriazin | 2,5 | 2 | | 1 | | |
| 4-Methoxyzimtsäure(2-ethylhexyl)-ester | 9,5 | 5 | | 2 | | |
| Octocrylene | | 7,5 | 5 | 3 | | 1 |
| Butyl Methoxydibenzoylmethan | 3 | | | 2,7 | 4,5 | |
| 2-(4'-Diethylamino-2'-hydoxybenzoyl)-benzoesäurehexylester | 1 | 2 | 5 | 1 | 2 | 7 |
| Phenylbenzimidazol Sulfonsäure | | | | 2,5 | 3 | |
| Methylpropandiol | 1 | 2 | | 3 | | 1 |
| Ölsäure | 0,5 | 1 | 0,8 | 2 | 4 | 0,1 |
| 1,2-Pentandiol | | 2 | 0,5 | 3 | 1 | |
| 1,2-Hexandiol | 0,2 | 0,1 | | 0,5 | 0,4 | |
| 1,2-Octandiol | | | 0,2 | | | 0,5 |
| Ethylhexylsalicylat | 2 | | 0,5 | 4 | 9,5 | |
| Bis-Ethylhexyloxyphenol Methoxyphenyltriazin | | 1 | | 1 | | 1 |
| Vitamin E Acetat | 0,2 | 0,2 | 0,2 | 0,3 | 0,1 | 0,5 |
| Na₂H₂EDTA | 0,1 | 0,1 | 0,2 | 0,2 | 0,2 | 0,5 |
| Parfüm, Konservierungsmittel | q.s. | q.s. | q.s. | q.s. | q.s. | q.s. |
| Farbstoffe, usw. | q.s. | q.s. | q.s. | q.s. | q.s. | q.s. |
| Citronensäure, Natriumcitrat | q.s. | q.s. | q.s. | q.s. | q.s. | q.s. |
| Natriumhydroxid | q.s. | q.s. | q.s. | q.s. | q.s. | q.s. |
| Wasser | ad 100,0 | ad 100,0 | ad 100,0 | ad 100,0 | ad 100,0 | ad 100,0 |

**Hydrodispersionen**

| | **1** | **2** | **3** | **4** | **5** | **6** |
|---|---|---|---|---|---|---|
| Glyceryl Stearat Citrat | | 0,40 | | | | |
| Sodium Carbomer | | | | | 0,30 | |
| Acrylates/C10-30 Alkyl Acrylate Crosspolymer | | | 0,30 | 0,40 | 0,10 | 0,10 |
| Ceteareth-20 | | | 1,00 | | | |
| Xanthan Gummi | 0,50 | | | 0,15 | | 0,50 |
| Dimethicon / Vinyl Dimethicon Crosspolymer | | | | 5,00 | | 3,00 |
| 2-(4'-Diethylamino-2'-hydoxybenzoyl)-benzoesäurehexylester | 0,25 | 5,00 | 7,00 | 0,50 | 2,00 | 1,50 |
| Bis-Ethylhexyloxyphenol Methoxhen Triazin | | 2,00 | | 0,25 | | |
| Ethylhexyl Methoxycinnamat | | | 7,00 | | 5,00 | 8,00 |
| Diethylhexyl Butamido Triazin | | | 2,00 | 1,00 | | |
| Ethylhexyl Triazin | 4,00 | 3,00 | | | 4,00 | |
| Titandioxid | 0,50 | 2,00 | 1,00 | 2,00 | 3,00 | 1,00 |
| Octocrylen | | 9,5 | 3 | 2 | | 7 |
| Butyl Methoxydibenzoylmethan | 4,2 | | 3,5 | | 4,5 | 1 |
| Methylpropandiol | 0,5 | 2 | 1,5 | 2,5 | 3 | 2 |
| C₁₂₋₁₅ Alkyl Benzoat | 2,00 | | 2,50 | | | |
| Ölsäure | 0,5 | 1 | 2 | 0,5 | 5 | 1 |
| C18-36 Triglycerid Fettsäure | | | 1,00 | | | |
| Butylenglycol Dicaprylat/Dicaprat | 4,00 | | | | 6,00 | |
| Dicaprylyl Carbonat | | 3,00 | | | | |
| Dicaprylylether | | 2,00 | | | | |
| Cyclomethicon | | | | 7,50 | | |
| PVP Hexadecen Copolymer | 0,50 | | 0,50 | | 0,50 | 1,00 |
| Glycerin | 10,00 | 5,00 | 5,00 | | 5,00 | 15,00 |
| Butylenglycol | | 7,00 | | | | |
| 1,2-Hexandiol | 0,50 | | | 1,00 | | 1,50 |
| 1,2-Octandiol | | 0,4 | 0,2 | | 0,1 | |
| Vitamin E Acetat | 0,50 | 0,25 | 0,50 | 0,25 | 0,75 | 1,00 |
| Panthenol | 1,50 | 0,50 | | | 0,25 | |
| Trinatrium EDTA | | 1,00 | 1,00 | 0,10 | 0,20 | |
| Ethanol | 3,00 | | 4,00 | 3,50 | | 1,00 |
| Konservierungsmittel | q.s. | q.s. | q.s. | q.s. | q.s. | q.s. |
| Parfüm, Farbstoffe, | q.s. | q.s. | q.s. | q.s. | q.s. | q.s. |
| Wasser | ad 100 | ad 100 | ad 100 | ad 100 | ad 100 | ad 100 |

**Gele**

| | **1** | **2** | **3** | **4** | **5** | **6** |
|---|---|---|---|---|---|---|
| Acrylat/Octylacrylamid Copolymer | 1,0 | 1,0 | 1,0 | 1,0 | 1,0 | 1,0 |
| Alkohol Denat. | 50,0 | 62,0 | 59,2 | 70,0 | 70,0 | 69,0 |
| Butylen Glycol Dicaprylat/Dicaprat | | | 9,5 | | | |
| C12-15 Alkyl Benzoat | 5,0 | 10,0 | 5,0 | 5,0 | 9,5 | |
| Phenylbenzoat | 5,0 | | | | | |
| Cocoglycerid | | | | | | 5 |
| Diethylamino Hydroxybenzoyl Hexyl Benzoat | 1,5 | 1 | 4,5 | 3,5 | 2,5 | 5 |
| Ethylhexyl Methoxycinnamat | 5 | 4,5 | | | 0,5 | |
| Ethylhexyl Salicylat | 4,5 | | 4,5 | 4,5 | | |
| Homosalat | | | | | | 4,5 |
| Hydroxypropylcellulose | 2 | 0,8 | 1 | 0,8 | 0,5 | 0,8 |
| Octocrylen | 7,5 | 9,5 | 3 | 2 | 2 | 3 |
| Butyl Methoxydibenzoylmethan | 4,2 | 4 | 3,5 | 2 | 4,5 | 3 |
| Methylpropandiol | 0,5 | 2 | 1,5 | 2,5 | 3 | 2 |
| 1,2-Hexandiol | | 0,3 | | 0,2 | | |
| 1,2-Octandiol | 0,4 | | 0,1 | 0,2 | 0,5 | 0,1 |
| Ethylhexyltriazin | | 2 | | | 2 | |
| Benzophenone-3 | 3 | | | | | |
| Bis-Ethylhexyloxyphenol Methoxyphenyltriazin | | | 2,5 | | 3 | 1 |
| Ölsäure | 0,5 | 1 | 2 | 1,5 | 0,5 | 3 |
| Vitamin E Acetat | | | | 0,5 | | 0,2 |
| Glycerin | 5 | | 3 | | | |
| Parfüm, Farbstoffe | q.s. | q.s. | q.s. | q.s. | q.s. | q.s. |
| Wasser | ad 100 | ad 100 | ad 100 | ad 100 | ad 100 | ad 100 |

| INCI | 1 | 2 | 3 |
|---|---|---|---|
| Methylparaben | - | 0,1 | 0,2 |
| Glyceryl Stearat | - | 3 | - |
| Caprlic/Capric Triglycerid | 1 | 1,5 | - |
| Wasser | ergänze zu 100 | ergänze zu 100 | ergänze zu 100 |
| Mikrokristallines Wachs | 0,7 | 1,5 | - |
| Parafinöl | 0,5 | 1 | - |
| Vitamin E Acetat | 0,5 | 0,5 | - |
| Cetearyl Alkohol | 4 | 4 | 1,5 |
| Cetyl Alkohol | 3 | - | 1,5 |
| Glyceryl Stearat SE | 2,6 | 2,6 | - |
| Trisodium EDTA | 1 | 1 | 1 |
| BHT | - | - | 0,01 |
| Ethylhexyl Methoxycinnamat | - | - | 7,5 |
| Phenoxyethanol | - | 0,4 | 0,4 |
| Dimethicon | 0,5 | 0,5 | 2 |
| Hydrogenierte Coco-Glyceride | 2 | - | 4 |
| C₁₂₋₁₅ Alkyl Benzoat | 1 | 2 | - |
| Butyl Methoxydibenzoylmethan | - | 3 | 2 |
| Phenylbenzimidazol Sulfonsäure | 1,5 | 1,4 | - |
| Glycerin | 8,7 | 8,7 | 9,7 |
| Natronlauge | pH-Einstellung | pH-Einstellung | pH-Einstellung |
| Butylene Glycol | - | - | 3 |
| Ethylhexyl Salicylate | 3,75 | - | 4,5 |
| Karietebutter | 3 | 3 | 2,5 |
| Ölsäure | 0,5 | 1 | 0,2 |
| Acrylats/C10-30 Alkyl Acrylat Crosspolymer | 0,1 | 0,1 | 0,1 |
| Titan Dioxid + Trimethoxycaprylylsilan | - | 0,5 | - |
| Glyceryl Stearat Citrat | - | - | 2 |
| Ethylhexylglycerin | 0,25 | 0,5 | 0,25 |
| Octocrylene | - | 3 | - |
| Pentylen Glycol | 1 | - | - |
| Chondrus Crispus | 0,2 | 0,2 | - |
| Bis-Ethylhexyloxyhenol Methoxyphenyl Triazin | - | 1,75 | 0,5 |
| Butylen Glycol Dicaprylat/Dicaprat | 3,5 | - | - |
| Methylpropandiol | 2 | 4 | 3 |
| Diethylamino Hydroxybenzoyl Hexyl Benzoate | 3 | 2 | 4,5 |
| 1,2-Hexandiol | 1 | - | - |
| Titan Dioxid + Aluminum Hydroxid + Dimethicon/Methicon Copolymer | 0,75 | - | 1 |
| Sodium Hyaluronat | 0,1 | 1 | 0,5 |
| Aqua + Pimpinella Anisum Extract | 5 | 2,5 | 4 |
| Parfum | 0,3 | 0,4 | 0,2 |
| Citronellol | 0,05 | 0,06 | 0,03 |

## Patentansprüche

1. Kosmetische Zubereitung enthaltend
a) 2-(4'-Diethylamino-2'-hydoxybenzoyl)-benzoesäurehexylester,
b) ein oder mehrere Diole und
c) Ölsäure, **dadurch gekennzeichnet, dass** die Zubereitung als weitere Komponenten eine oder mehrere Verbindungen gewählt aus der Gruppe der Verbindungen Limonen, Linalool, Hexylcinnamal, Benzylsalicylat, Citral, alpha-Isomethylionon, Geraniol, Methyl-2-Octynoat, Citronellol, Isoeugenol, 2-Isobutyl-4-hydroxy-4-methyltetrahydropyran, 2-tert-Pentylcyclohexylacetat, 3-Methyl-5-phenyl-1-pentanol, 7-Acetyl-1,1,3,4,4,6-hexamethyltetralin, Adipinsäurediester, alpha-Amylcinnamaldehyd, Alpha-Methylionon, Amyl C Butylphenylmethylpropionalcinnamal, Amylsalicylat, Amylcinnamylalkohol, Anisalkohol, Benzoin, Benzylalkohol, Benzylbenzoat, Benzylcinnamat, Bergamotöl, bitteres Orangenöl, Butylphenylmethylpropioal, Cardamomöl, Cedrol, Cinnamal, Cinnamylalkohol, Citronellylmethylcrotonat, Citronenöl, Coumarin, Diethylsuccinat, d-Limonene, Ethyllinalool, Eugenol, Evernia Furfuracea Extract, Evernia Prunastri Extract, Farnesol, Guajakholzöl, Hexylsalicylat, Hydroxycitronellal, Hydroxyisohexyl 3-Cyclohexencarboxaldehyde, Lavendelöl, Lemonenöl, Linaylacetat, Mandarinenöl, Menthyl PCA, Methylheptenon, Muskatnussöl, Rosmarinöl, süßes Orangenöl, Terpineol, Tonkabohnenöl, Triethylcitrat und/oder Vanillin enthält.

2. Verwendung von 2-(4'-Diethylamino-2'-hydoxybenzoyl)-benzoesäurehexylester zur Unterdrückung des Eigengeruches von Diolen und Ölsäure in kosmetischen Zubereitungen.

3. Kosmetische Zubereitung nach Anspruch 1 oder Verwendung nach Anspruch 2, **dadurch gekennzeichnet, dass** ein oder mehrere Diole gewählt werden aus der Gruppe der Verbindungen 2-Methyl-1,3-propandiol, Pentan-1,2-diol, Hexan-1,2-diol, Heptan-1,2-diol, Octan-1,2-diol, Nonan-1,2-diol, Decan-1,2-diol.

4. Kosmetische Zubereitung oder Verwendung nach einem der vorhergehenden Ansprüche, **dadurch gekennzeichnet, dass** die Zubereitung 2-(4'-Diethylamino-2'-hydoxybenzoyl)-benzoesäurehexylester in einer Konzentration von 0,1 bis 10 Gewichts-%, bezogen auf das Gesamtgewicht der Zubereitung, enthält.

5. Kosmetische Zubereitung oder Verwendung nach einem der vorhergehenden Ansprüche, **dadurch gekennzeichnet, dass** die Zubereitung einen oder mehrere Farbstoffe enthält.

6. Kosmetische Zubereitung oder Verwendung nach einem der vorhergehenden Ansprüche, **dadurch gekennzeichnet, dass** die Zubereitung anorganische Farbpigmente, insbesondere Titandioxid, Eisenoxide und/oder Bariumsulfat enthält.

7. Kosmetische Zubereitung oder Verwendung nach einem der vorhergehenden Ansprüche, **dadurch gekennzeichnet, dass** die Zubereitung ein oder mehrere Diole in einer Gesamtkonzentration von 0,1 bis 5 Gewichts-%, bezogen auf das Gesamtgewicht der Zubereitung, enthält.

8. Kosmetische Zubereitung oder Verwendung nach einem der vorhergehenden Ansprüche, **dadurch gekennzeichnet, dass** die Zubereitung Ölsäure in einer Konzentration von 0,1 bis 10 Gewichts-%, bezogen auf das Gesamtgewicht der Zubereitung, enthält.

9. Kosmetische Zubereitung oder Verwendung nach einem der vorhergehenden Ansprüche, **dadurch gekennzeichnet, dass** die Zubereitung einen oder mehrere Farbstoffe gewählt aus der Gruppe der Farbstoffe mit dem Color-Index CI16035 und CI 15985 enthält.

10. Kosmetische Zubereitung oder Verwendung nach einem der vorhergehenden Ansprüche, **dadurch gekennzeichnet, dass** die Zubereitung einen oder mehrere Farbstoffe in einer Gesamtkonzentration von 0,001 bis 1,0 Gewichts-%, bezogen auf das Gesamtgewicht der Zubereitung, enthält.

11. Verwendung nach einem der vorhergehenden Ansprüche, **dadurch gekennzeichnet, dass** die Zubereitung als weitere Komponenten eine oder mehrere Verbindungen gewählt aus der Gruppe der Verbindungen
Limonen, Linalool, Hexylcinnamal, Benzylsalicylat, Citral, alpha-Isomethylionon, Geraniol, Methyl-2-Octynoat, Citronellol, Isoeugenol, 2-Isobutyl-4-hydroxy-4-methyltetrahydropyran, 2-tert-Pentylcyclohexylacetat, 3-Methyl-5-phenyl-1-pentanol, 7-Acetyl-1,1,3,4,4,6-hexamethyltetralin, Adipinsäurediester, alpha-Amylcinnamaldehyd, Alpha-Methylionon, Amyl C Butylphenylmethylpropionalcinnamal, Amylsalicylat, Amylcinnamylalkohol, Anisalkohol, Benzoin, Benzylalkohol, Benzylbenzoat, Benzylcinnamat, Bergamotöl, bitteres Orangenöl, Butylphenylmethylpropioal, Cardamomöl, Cedrol, Cinnamal, Cinnamylalkohol, Citronellylmethylcrotonat, Citronenöl, Coumarin, Diethylsuccinat, d-Limonene, Ethyllinalool, Eugenol, Evernia Furfuracea Extract, Evernia Prunastri Extract, Farnesol, Guajakholzöl, Hexylsalicylat, Hydroxycitronellal, Hydroxyisohexyl 3-Cyclohexencarboxaldehyde, Lavendelöl, Lemonenöl, Linaylacetat, Mandarinenöl, Menthyl PCA, Methylheptenon, Muskatnussöl, Rosmarinöl, süßes Orangenöl, Terpineol, Tonkabohnenöl, Triethylcitrat und/oder Vanillin enthält.

12. Kosmetische Zubereitung oder Verwendung nach einem der vorhergehenden Ansprüche, **dadurch gekennzeichnet, dass** die Zubereitung in Form einer Emulsion oder Dispersion vorliegt.

13. Kosmetische Zubereitung oder Verwendung nach einem der vorhergehenden Ansprüche, **dadurch gekennzeichnet, dass** die Zubereitung einen oder mehrere weitere UV-Filter enthält, gewählt aus der Gruppe der Verbindungen Phenylen-1,4-bis-(2-benzimidazyl)-3,3'-5,5'-tetrasulfonsäuresalze; 2-Phenylbenzimidazol-5-sulfonsäuresalze; 1,4-di(2-oxo-10-Sulfo-3-bornylidenmethyl)-Benzol und dessen Salze; 4-(2-Oxo-3-bornylidenmethyl)benzolsulfonsäuresalze; 2-Methyl-5-(2-oxo-3-bornylidenmethyl)sulfonsäuresalze; 2,2'-Methylen-bis-(6-(2H-benzotriazol-2-yl)-4-(1,1,3,3-tetramethylbutyl)-phenol); 2-(2H-benzotriazol-2-yl)-4-methyl-6-[2-methyl-3-[1,3,3,3-tetramethyl-1-[(trimethylsilyl)oxy]disiloxanyl]propyl]-phenol; 3-(4-Methylbenzyliden)campher; 3-Benzylidencampher; Ethylhexylsalicylat;
Terephthalidendicamphersulfonsäure; 4-(Dimethylamino)-benzoesäure(2-ethylhexyl)-ester; 4-(Dimethylamino)benzoesäure-amylester; 4-Methoxybenzalmalon-säuredi(2-ethylhexyl)ester; 4-Methoxyzimtsäure(2-ethylhexyl)ester; 4-Methoxyzimtsäureisoamylester; 2-Hydroxy-4-methoxybenzophenon, 2-Hydroxy-4-methoxy-4'-methylbenzophenon; 2,2'-Dihydroxy-4-methoxybenzophenon; 4-(tert.-Butyl)-4'-methoxydibenzoylmethan; Homomenthylsalicylat; 2-Ethylhexyl-2-hydroxybenzoat; 2-Ethylhexyl-2-cyano-3,3-diphenylacrylat; Dimethicodiethylbenzalmalonat; 3-(4-(2,2-bis Ethoxycarbonylvinyl)-phenoxy)propenyl)-methoxysiloxan / Dimethylsiloxan - Copolymer; Dioctylbutylamidotriazon (INCI: Diethylhexyl-Butamidotriazone); 2,4-bis-[5-1(dimethylpropyl)benzoxazol-2-yl-(4-phenyl)-imino]-6-(2-ethylhexyl)-imino-1,3,5-triazin mit der (CAS Nr. 288254-16-0); 4,4',4"-(1,3,5-Triazin-2,4,6-triyltriimino)-tris-benzoesäure-tris(2-ethylhexylester) (auch: 2,4,6-Tris-[anilino-(p-carbo-2'-ethyl-1'-hexyloxy)]-1,3,5-triazin (INCI: Ethylhexyl Triazone); 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazin); 2,4,6-Tris-(biphenyl)-1,3,5-triazin; 2,4-Bis-(4'- Di-neopentylaminobenzalmalonat)-6-(4"-butylaminobenzoat)-s-triazin Titandioxid, Zinkoxid, Merocyanine gewählt aus der Gruppe der Verbindungen

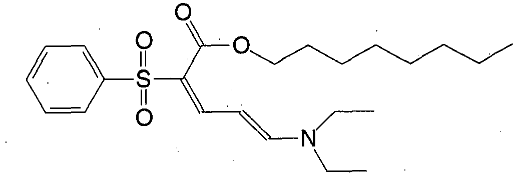

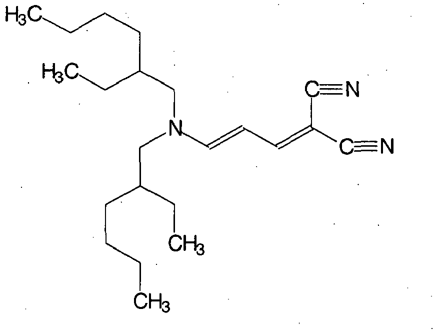

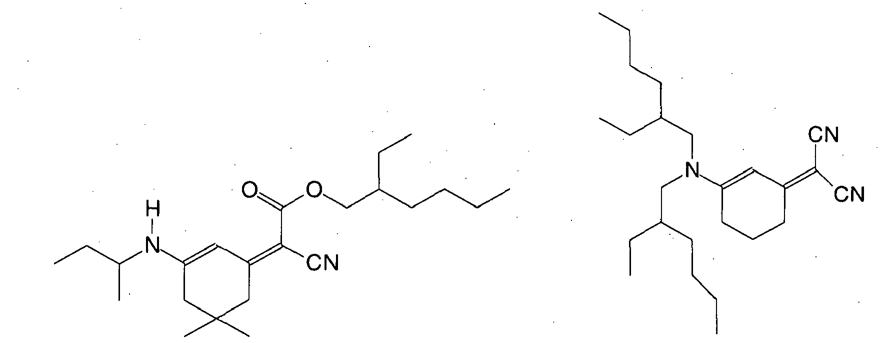

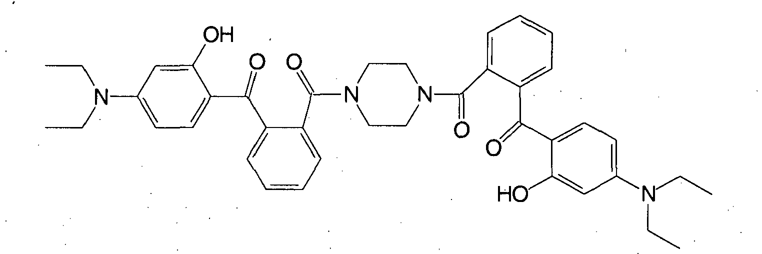

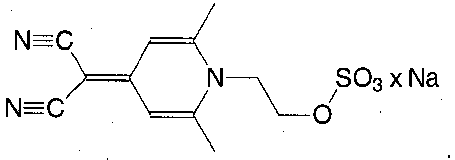

14. Kosmetische Zubereitung oder Verwendung nach einem der vorhergehenden Ansprüche, **dadurch gekennzeichnet, dass** die Zubereitung als weitere Inhaltsstoffe eine oder mehrere Verbindungen gewählt aus der Gruppe der Verbindungen alpha-Liponsäure, Folsäure, Phytoen, D-Biotin, Coenzym Q10, alpha-Glucosylrutin, Carnitin, Carnosin, natürliche und/oder synthetische Isoflavonoide, Flavonoide, Kreatin, Kreatinin, Taurin, β-Alanin, Tocopherylacetat, Dihydroxyaceton; 8-Hexadecen-1,16-dicarbonsäure, Glycerylglycose, (2-Hydroxyethyl)harnstoff, Niacinamid, Vitamin E bzw. seine Derivate und/oder Licochalcon A, enthält.

15. Kosmetische Zubereitung oder Verwendung nach einem der vorhergehenden Ansprüche, **dadurch gekennzeichnet, dass** die Zubereitung einen pH-Wert von 5 bis 8 aufweist.

16. Kosmetische Zubereitung oder Verwendung nach einem der Ansprüche 1 bis 12, 14 und 15, **dadurch gekennzeichnet, dass** die Zubereitung keine UV-B-Filter enthält.

## Claims

1. Cosmetic formulation comprising
a) hexyl 2-(4'-diethylamino-2'-hydroxybenzyl)-benzoate,
b) one or more diols and
c) oleic acid, **characterized in that** the formulation comprises, as further components, limonene, linalool, hexylcinnamal, benzyl salicylate, citral, alpha-isomethylionone, geraniol, methyl 2-octynoate, citronellol, isoeugenol, 2-isobutyl-4-hydroxy-4-methyltetra-hydropyran, 2-tert-pentylcyclohexyl acetate, 3-methyl-5-phenyl-1-pentanol, 7-acetyl-1,1,3,4,4,6-hexamethyltetralin, adipic diesters, alpha-amylcinnamaldehyde, alpha-methylionone, amyl C butylphenylmethylpropionalcinnamal, amyl salicylate, amylcinnamyl alcohol, anise alcohol, benzoin, benzyl alcohol, benzyl benzoate, benzyl cinnamate, bergamot oil, bitter orange oil, butylphenyl methylpropional, cardamom oil, cedrol, cinnamal, cinnamyl alcohol, citronellylmethyl crotonate, lemon oil, coumarin, diethyl succinate, d-limonene, ethyllinalool, eugenol, Evernia furfuracea extract, Evernia prunastri extract, farnesol, guaiac wood oil, hexyl salicylate, hydroxycitronellal, hydroxyisohexyl 3- cyclohexenecarboxaldehyde, lavender oil, lime oil, linayl acetate, mandarin oil, menthyl PCA, methylheptenone, nutmeg oil, rosemary oil, sweet orange oil, terpineol, tonka bean oil, triethyl citrate and/or vanillin.

2. Use of hexyl 2-(4'-diethylamino-2'-hydroxybenzyl)-benzoate for suppression of the intrinsic odor of diols and oleic acid in cosmetic formulations.

3. Cosmetic formulation according to Claim 1 or use according to Claim 2, **characterized in that** one or more diols are selected from the group of compounds 2-methylpropane-1,3-diol, pentane-1,2-diol, hexane-1,2-diol, heptane-1,2-diol, octane-1,2-diol, nonane-1,2-diol, decane-1,2-diol.

4. Cosmetic formulation or use according to any of the preceding claims, **characterized in that** the formula contains hexyl 2-(4'-diethylamino-2'-hydroxy-benzyl)benzoate in a concentration of 0.1% to 10% by weight, based on the total weight of the formulation.

5. Cosmetic formulation or use according to any of the preceding claims, **characterized in that** the formulation comprises one or more dyes.

6. Cosmetic formulation or use according to any of the preceding claims, **characterized in that** the formulation comprises inorganic colour pigments, especially titanium dioxide, iron oxides and/or barium sulfate.

7. Cosmetic formulation or use according to any of the preceding claims, **characterized in that** the formulation comprises one or more diols in a total concentration of 0.1% to 5% by weight, based on the total weight of the formulation.

8. Cosmetic formulation or use according to any of the preceding claims, **characterized in that** the formulation contains oleic acid in a concentration of 0.1% to 10% by weight, based on the total weight of the formulation.

9. Cosmetic formulation or use according to any of the preceding claims, **characterized in that** the formulation comprises one or more dyes are selected from the group of the dyes having Color Index CI 16035 and CI 15985.

10. Cosmetic formulation or use according to any of the preceding claims, **characterized in that** the formulation contains one or more dyes in a total concentration of 0.001% to 1.0% by weight, based on the total weight of the formulation.

11. Cosmetic formulation or use according to any of the preceding claims, **characterized in that** the formulation comprises, as further components, one or more compounds selected from the group of the compounds limonene, linalool, hexylcinnamal, benzyl salicylate, citral, alpha-isomethylionone, geraniol, methyl 2-octynoate, citronellol, isoeugenol, 2-isobutyl-4-hydroxy-4-methyltetra-hydropyran, 2-tert-pentylcyclohexyl acetate, 3-methyl-5-phenyl-1-pentanol, 7-acetyl-1,1,3,4,4,6-hexamethyltetralin, adipic diesters, alpha-amylcinnamaldehyde, alpha-methylionone, amyl C butylphenylmethylpropionalcinnamal, amyl salicylate, amylcinnamyl alcohol, anise alcohol, benzoin, benzyl alcohol, benzyl benzoate, benzyl cinnamate, bergamot oil, bitter orange oil, butylphenyl methylpropional, cardamom oil, cedrol, cinnamal, cinnamyl alcohol, citronellylmethyl crotonate, lemon oil, coumarin, diethyl succinate, d-limonene, ethyllinalool, eugenol, Evernia furfuracea extract, Evernia prunastri extract, farnesol, guaiac wood oil, hexyl salicylate, hydroxycitronellal, hydroxyisohexyl 3- cyclohexenecarboxaldehyde, lavender oil, lime oil, linayl acetate, mandarin oil, menthyl PCA, methylheptenone, nutmeg oil, rosemary oil, sweet orange oil, terpineol, tonka bean oil, triethyl citrate and/or vanillin.

12. Cosmetic formulation or use according to any of the preceding claims, **characterized in that** the formulation takes the form of an emulsion or dispersion.

13. Cosmetic formulation or use according to any of the preceding claims, **characterized in that** the formulation comprises one or more further UV filters selected from the group of the compounds phenylene-1,4-bis-(2-benzimidazyl)-3,3':5,5'-tetrasulfonic acid salts; 2-phenylbenzimidazole-5-sulfonic acid salts; 1,4-di(2-oxo-10-sulfo-3-bornylidenemethyl)benzene and salts thereof; 4-(2-oxo-3-bornylidenemethyl)benzenesulfonic acid salts; 2-methyl-5-(2-oxo-3-bornylidenemethyl)sulfonic acid salts; 2,2'-methylenebis(6-(2H-benzotriazol-2-yl)-4-(1,1,3,3-tetramethylbutyl)phenol); 2-(2H-benzotriazol-2-yl)-4-methyl-6-[2-methyl-3-[1,3,3,3-tetramethyl-1-[(trimethylsilyl)oxy]disiloxanyl]-propyl]phenol; 3-(4-methylbenzyliden)camphor; 3-benzylidencamphor; ethylhexyl salicylate; terephthalidenedicamphorsulfonic acid; 2-ethylhexyl 4-(dimethylamino)benzoate; amyl 4-(dimethylamino)-benzoate; di(2-ethylhexyl) 4-methoxybenzalmalonate; 2-ethylhexyl 4-methoxycinnamate; isoamyl 4-methoxycinnamate; 2-hydroxy-4-methoxybenzophenone; 2-hydroxy-4-methoxy-4'-methylbenzophenone; 2,2'-dihydroxy-4-methoxybenzophenone; 4-(tert-butyl)-4'-methoxydibenzoylmethane; homomenthyl salicylate; 2-ethylhexyl 2-hydroxybenzoate; 2-ethylhexyl 2-cyano-3,3-diphenyl acrylate; dimethicodiethyl benzalmalonate; 3-(4-(2,2-bisethoxycarbonylvinyl)-phenoxy)propenyl)methoxysiloxane/dimethylsiloxane copolymer; dioctylbutylamidotriazone (INCI: Diethylhexyl-Butamidotriazone); 2,4-bis[5-1(dimethylpropyl)benzoxazol-2-yl-(4-phenyl)imino]-6-(2-ethylhexyl)imino-1,3,5-triazine with (CAS No. 288254-16-0); tris(2-ethylhexyl) 4,4',4"-(1,3,5-triazine-2,4,6-triyltriimino)trisbenzoate (also: 2,4,6-tris[anilino-(p-carbo-2'-ethyl-1'-hexyloxy)]-1,3,5-triazine (INCI: Ethylhexyl Triazone); 2,4-bis-{[4-(2-ethylhexyloxy)-2-hydroxy]phenyl}-6-(4-methoxyphenyl)-1,3,5-triazine (INCI: Bis-Ethylhexyloxyphenol Methoxyphenyl Triazine); 2,4,6-tris(biphenyl)-1,3,5-triazine; 2,4-bis(4'-dineopentylaminobenzalmalonat)-6-(4"-butylamino-benzoate)-s-triazine titanium dioxide, zinc oxide, merocyanines selected from the group of the compounds

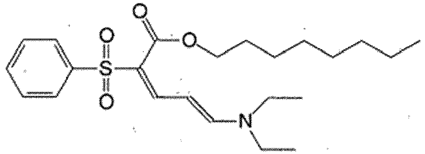

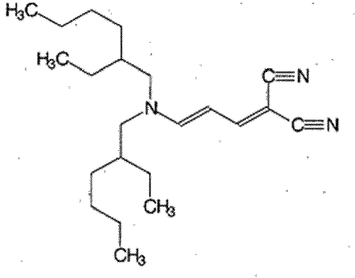

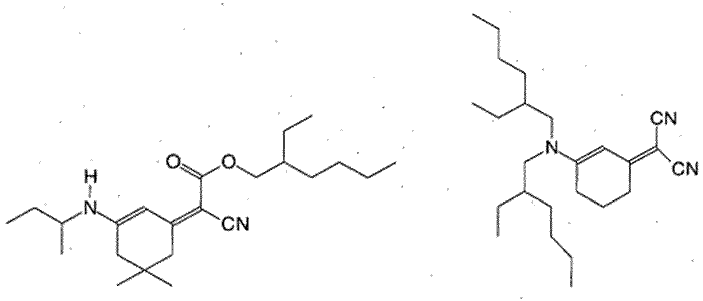

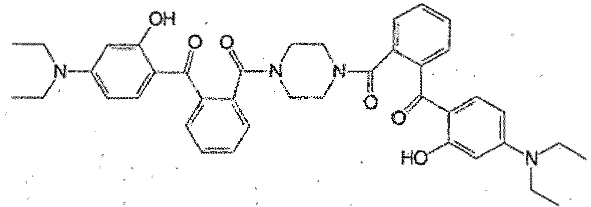

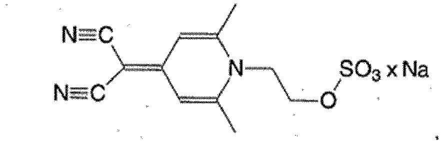

14. Cosmetic formulation or use according to any of the preceding claims, **characterized in that** the formulation comprises, as further ingredients, one or more compounds selected from the group of the compounds alpha-lipoic acid, folic acid, phytoene, D-biotin, coenzyme Q10, alpha-glucosylrutine, carnitine, carnosine, natural and/or synthetic isoflavonoids, flavonoids, creatine, creatinine, taurine, β-alanine, tocopheryl acetate, dihydroxyacetone; 8-hexadecene-1,16-dicarboxylic acid, glycerylglycose, (2-hydroxyethyl)urea, niacinamide, vitamin E or derivatives thereof and/or licochalcone A.

15. Cosmetic formulation or use according to any of the preceding claims, **characterized in that** the formulation has a pH of 5 to 8.

16. Cosmetic formulation or use according to any of Claims 1 to 12, 14 and 15, **characterized in that** the formulation does not contain any UV-B filters.

## Revendications

1. Préparation cosmétique contenant
a) de l'ester hexylique de l'acide 2-(4'-diéthylamino-2'-hydoxybenzoyl)-benzoïque,
b) un ou plusieurs diols et
c) de l'acide oléique, **caractérisée**
**en ce que** la préparation contient comme autres composants un ou plusieurs composés du groupe formé par les composés limonène, linalool, hexylcinnamal, salicylate de benzyle, citral, alpha-isométhylionone, géraniol, 2-octynoate de méthyle, citronellol, iso-eugénol, 2-isobutyl-4-hydroxy-4-méthyltétrahydropyrane, acétate de 2-tert-pentylcyclohexyle, 3-méthyl-5-phényl-1-pentanol, 7-acétyl-1,1,3,4,4,6-hexaméthyltétraline, diester de l'acide adipique, alpha-amylcinnamaldéhyde, alpha-méthylionone, Amyl C, butylphénylméthylpropionalcinnamal, salicylate d'amyle, alcool amylcinnamylique, alcool anisique, benzoïne, alcool benzylique, benzoate de benzyle, cinnamate de benzyle, huile de bergamote, huile d'orange amère, butylphénylméthylpropioal, huile de cardamome, cédrol, cinnamal, alcool cinnamylique, crotonate de citronellylméthyle, huile de citron, coumarine, succinate de diéthyle, d-limonène, éthyllinalool, eugénol, extrait d'Evernia Furfuracea, extrait d'Evernia Prunastri, farnésol, huile de bois de gaïac, salicylate d'hexyle, hydroxycitronellal, hydroxyisohexyl-3-cyclohexènecarboxaldéhyde, huile de lavande, huile de limonène, acétate de linayle, huile de mandarine, menthyl-PCA, méthylhepténone, huile de noix de muscade, huile de romarin, huile d'orange douce, terpinéol, huile de fèves de tonka, citrate de triéthyle et/ou vanilline.

2. Utilisation de l'ester hexylique de l'acide 2-(4'-diéthylamino-2'-hydoxybenzoyl)-benzoïque pour supprimer l'odeur propre de diols et de l'acide oléique dans des préparations cosmétiques.

3. Préparation cosmétique selon la revendication 1 ou utilisation selon la revendication 2, **caractérisée en ce qu'**un ou plusieurs diols sont choisis dans le groupe des composés 2-méthyl-1,3-propanediol, pentane-1,2-diol, hexane-1,2-diol, heptane-1,2-diol, octane-1,2-diol, nonane-1,2-diol, décane-1,2-diol.

4. Préparation cosmétique ou utilisation selon l'une quelconque des revendications précédentes, **caractérisée en ce que** la préparation contient l'ester hexylique de l'acide 2-(4'-diéthylamino-2'-hydoxybenzoyl)-benzoïque en une concentration de 0,1 à 10% en poids, par rapport au poids total de la préparation.

5. Préparation cosmétique ou utilisation selon l'une quelconque des revendications précédentes, **caractérisée en ce que** la préparation contient un ou plusieurs colorants.

6. Préparation cosmétique ou utilisation selon l'une quelconque des revendications précédentes, **caractérisée en ce que** la préparation contient des pigments colorés inorganiques, en particulier le dioxyde de titane, les oxydes de fer et/ou le sulfate de baryum.

7. Préparation cosmétique ou utilisation selon l'une quelconque des revendications précédentes, **caractérisée en ce que** la préparation contient un ou plusieurs diols en une concentration totale de 0,1 à 5% en poids, par rapport au poids total de la préparation.

8. Préparation cosmétique ou utilisation selon l'une quelconque des revendications précédentes, **caractérisée en ce que** la préparation contient l'acide oléique en une concentration de 0,1 à 10% en poids, par rapport au poids total de la préparation.

9. Préparation cosmétique ou utilisation selon l'une quelconque des revendications précédentes, **caractérisée en ce que** la préparation contient un ou plusieurs colorants choisis dans le groupe des colorants présentant le Color-Index CI16035 et CI15985.

10. Préparation cosmétique ou utilisation selon l'une quelconque des revendications précédentes, **caractérisée en ce que** la préparation contient un ou plusieurs colorants en une concentration totale de 0,001 à 1,0% en poids, par rapport au poids total de la préparation.

11. Utilisation selon l'une quelconque des revendications précédentes, **caractérisée en ce que** la préparation contient comme autres composants un ou plusieurs composés du groupe formé par les composés limonène, linalool, hexylcinnamal, salicylate de benzyle, citral, alpha-isométhylionone, géraniol, 2-octynoate de méthyle, citronellol, iso-eugénol, 2-isobutyl-4-hydroxy-4-méthyltétrahydropyrane, acétate de 2-tert-pentylcyclohexyle, 3-méthyl-5-phényl-1-pentanol, 7-acétyl-1,1,3,4,4,6-hexaméthyltétraline, diester de l'acide adipique, alpha-amylcinnamaldéhyde, alpha-méthylionone, Amyl C, butylphénylméthylpropionalcinnamal, salicylate d'amyle, alcool amylcinnamylique, alcool anisique, benzoïne, alcool benzylique, benzoate de benzyle, cinnamate de benzyle, huile de bergamote, huile d'orange amère, butylphénylméthylpropioal, huile de cardamome, cédrol, cinnamal, alcool cinnamylique, crotonate de citronellylméthyle, huile de citron, coumarine, succinate de diéthyle, d-limonène, éthyllinalool, eugénol, extrait d'Evernia Furfuracea, extrait d'Evernia Prunastri, farnésol, huile de bois de gaïac, salicylate d'hexyle, hydroxycitronellal, hydroxyisohexyl-3-cyclohexènecarboxaldéhyde, huile de lavande, huile de limonène, acétate de linayle, huile de mandarine, menthyl-PCA, méthylhepténone, huile de noix de muscade, huile de romarin, huile d'orange douce, terpinéol, huile de fèves de tonka, citrate de triéthyle et/ou vanilline.

12. Préparation cosmétique ou utilisation selon l'une quelconque des revendications précédentes, **caractérisée en ce que** la préparation se trouve sous forme d'une émulsion ou d'une dispersion.

13. Préparation cosmétique ou utilisation selon l'une quelconque des revendications précédentes, **caractérisée en ce que** la préparation contient un ou plusieurs autres filtres UV choisis dans le groupe des composés : sels de l'acide phénylène-1,4-bis-(2-benzimidazyl)-3,3'-5,5'-tétrasulfonique ; sels de l'acide 2-phénylbenzimidazole-5-sulfonique ; 1,4-di(2-oxo-10-sulfo-3-bornylidèneméthyl)-benzène et ses sels ; sels de l'acide 4-(2-oxo-3-bornylidèneméthyl)benzènesulfonique ; sels de l'acide 2-méthyl-5-(2-oxo-3-bornylidèneméthyl)sulfonique ; 2,2'-méthylènebis-(6-(2H-benzotriazol-2-yl)-4-(1,1,3,3-tétraméthylbutyl)-phénol) ; 2-(2H-benzotriazol-2-yl)-4-méthyl-6-[2-méthyl-3-(1,3,3,3-tétraméthyl-1-[(triméthylsilyl)oxy]disiloxanyl]propyl]-phénol ; 3-(4-méthylbenzylidène)camphre ; 3-benzylidènecamphre ; salicylate d'éthylhexyle ; acide téréphtalidènedicamphresulfonique ; ester 2-éthylhexylique de l'acide 4-(diméthylamino)-benzoïque ; ester amylique de l'acide 4-(diméthylamino)benzoïque ; ester di(2-éthylhexylique) de l'acide 4-méthoxybenzalmalonique ; ester 2-éthylhexylique de l'acide 4-méthoxycinnamique ; ester isoamylique de l'acide 4-méthoxycinnamique ; 2-hydroxy-4-méthoxybenzophénone, 2-hydroxy-4-méthoxy-4'-méthylbenzophénone ; 2,2'-dihydroxy-4-méthoxybenzophénone ; 4-(tert-butyl)-4'-méthoxydibenzoylméthane ; salicylate d'homomenthyle ; 2-hydroxybenzoate de 2-éthylhexyle ; 2-cyano-3,3-diphénylacrylate de 2-éthylhexyle ; benzalmalonate de diméthicodiéthyle ; copolymère de 3-(4-(2,2-bis(éthoxycarbonylvinyl)-phénoxy)propényl)-méthoxysiloxane/diméthylsiloxane ; dioctylbutylamidotriazone (INCI : Diethylhexyl-Butamidotriazone) ; 2,4-bis-[5-1(diméthylpropyl)benzoxazol-2-yl-(4-phényl)-imino]-6-(2-éthylhexyl)-imino-1,3,5-triazine présentant le (n° CAS 285254-16-0 ; ester tris(2-éthylhexylique) de l'acide 4,4',4"-(1,3,5-triazine-2,4,6-triyltriimino)-trisbenzoïque (également : 2,4,6-tris-[anilino-(p-carbo-2'-éthyl-1'-hexyloxy)]-1,3,5-triazine INCI : Ethylhexyl Triazone) ; 2,4-bis-{[4-(2-éthylhexyloxy)-2-hydroxy]-phényl}-6-(4-méthoxyphényl)-1,3,5-triazine (INCI : Bis-Ethylhexyloxyphenol Methoxyphenyl Triazin) ; 2,4,6-tris-(biphényl)-1,3,5-triazine ; 2,4-bis-(4'-dinéopentylaminobenzalmalonate)-6-(4"-butylaminobenzoate)-s-triazine dioxyde de titane, oxyde de zinc, mérocyanines choisies dans le groupe de composés

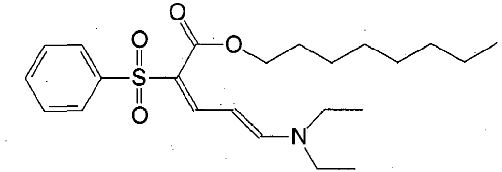

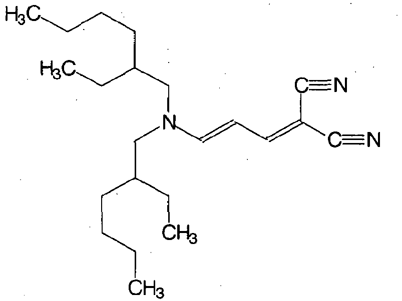

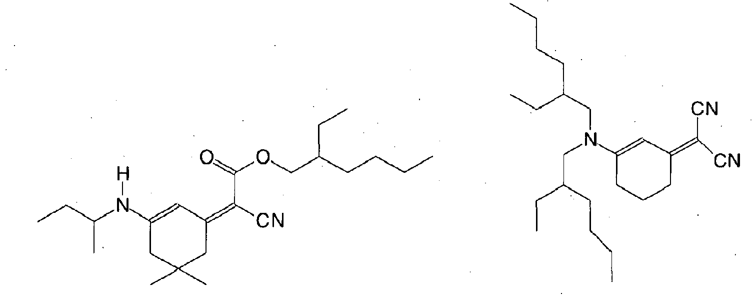

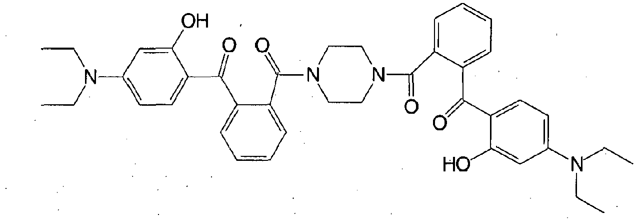

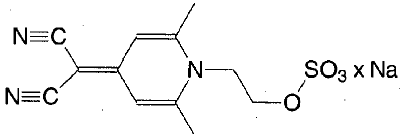

14. Préparation cosmétique ou utilisation selon l'une quelconque des revendications précédentes, **caractérisée en ce que** la préparation contient, comme autres constituants, un ou plusieurs composés choisis dans le groupe des composés acide alpha-liponique, acide folique, phytoène, D-biotine, coenzyme Q10, alpha-glucosylrutine, carnitine, carnosine, isoflavonoïdes naturels ou synthétiques, flavonoïdes, créatine, créatinine, taurine, β-alanine, acétate de tocophéryle, dihydroxyacétone ; acide 8-hexadécène-1,16-dicarboxylique, glycérylglucose, (2-hydroxyéthyl)urée, niacinamide, vitamine E ou, selon le cas, ses dérivés et/ou licochalcone A.

15. Préparation cosmétique ou utilisation selon l'une quelconque des revendications précédentes, **caractérisée en ce que** la préparation présente un pH de 5 à 8.

16. Préparation cosmétique ou utilisation selon l'une quelconque des revendications 1 à 12, 14 et 15, **caractérisée en ce que** la préparation ne contient pas de filtres UV-B.
